# EUROPEAN PATENT SPECIFICATION

(11) **EP 3 478 305 B1**
(45) Date of publication and mention of the grant of the patent: **08.07.2026**
(21) Application number: 17732481.1
(22) Date of filing: 29.06.2017
(51) Int. Cl.: A61K 35/744, A61K 35/745, A61K 35/747, A23L 33/00, A61P 25/22

(54) **NUTRITIONAL COMPOSITION COMPRISING A PROBIOTIC FOR THE PREVENTION OF ANXIETY DISORDERS IN A MAMMAL**
NÄHRSTOFFZUSAMMENSETZUNG MIT EINEM PROBIOTIKUM ZUR PRÄVENTION VON ANGSTZUSTÄNDEN IN EINEM SÄUGETIER
COMPOSITION NUTRITIONNELLE COMPRENANT UN PROBIOTIQUE POUR LA PRÉVENTION DE TROUBLES DE L'ANXIÉTÉ CHEZ UN MAMMIFÈRE

(30) Priority: 01.07.2016 EP 16177649
(43) Date of publication of application: 08.05.2019
(73) Proprietor: Société des Produits Nestlé S.A., 1800 Vevey (CH)
(72) Inventor: SCHNEIDER, Nora, 1066 Epalinges Vaud (CH); MITCHELL, Ellen, Siobhan, White Plains, NY 10605 (US); BLANCHARD, Carine, 1052 Le Mont-sur-Lausanne (CH); NEMBRINI, Chiara, 1081 MONTPREVEYRES (CH)
(74) Representative: Ng, Yen Kar
(86) International application number: PCT/EP2017/066161
(87) International publication number: WO 2018/002240

(56) References cited:
- EP-A1- 2 110 028
- WO-A1-2015/086789
- WO-A1-2017/037105
- US-A1- 2011 027 348
- US-A1- 2014 363 410
- US-A1- 2015 305 384
- US-A1- 2016 058 808
- LAURA STEENBERGEN ET AL: "A randomized controlled trial to test the effect of multispecies probiotics on cognitive reactivity to sad mood", BRAIN, BEHAVIOR AND IMMUNITY., vol. 48, 1 August 2015 (2015-08-01), US, pages 258 - 264, XP055226948, ISSN: 0889-1591, DOI: 10.1016/j.bbi.2015.04.003
- BERCIK P ET AL: "Chronic Gastrointestinal Inflammation Induces Anxiety-Like Behavior and Alters Central Nervous System Biochemistry in Mice", GASTROENTEROLOGY, ELSEVIER, AMSTERDAM, NL, vol. 139, no. 6, 1 December 2010 (2010-12-01), pages 2102 - 2112.e1, XP027523611, ISSN: 0016-5085, [retrieved on 20101130]
- KANTAK PRANISH A ET AL: "Obsessive-compulsive-like behaviors in house mice are attenuated by a probiotic (Lactobacillus rhamnosus GG)", BEHAVIOURAL PHARMACOLOGY, vol. 25, no. 1, February 2014 (2014-02-01), pages 71 - 79, XP021175131
- SAMPSON TIMOTHY R ET AL: "Control of Brain Development, Function, and Behavior by the Microbiome", CELL HOST & MICROBE, vol. 17, no. 5, 13 May 2015 (2015-05-13), pages 565 - 576, XP029169456, ISSN: 1931-3128, DOI: 10.1016/J.CHOM.2015.04.011
- R. M. STILLING ET AL: "Microbial genes, brain & behaviour - epigenetic regulation of the gut-brain axis", GENES BRAIN AND BEHAVIOR, vol. 13, no. 1, 27 January 2014 (2014-01-27), DK, pages 69 - 86, XP055330714, ISSN: 1601-1848, DOI: 10.1111/gbb.12109
- BAROUEI J ET AL: "Prophylactic role of maternal administration of probiotics in the prevention of irritable bowel syndrome", MEDICAL HYPOTHESES, EDEN PRESS, PENRITH, US, vol. 73, no. 5, 1 November 2009 (2009-11-01), pages 764 - 767, XP026680495, ISSN: 0306-9877, [retrieved on 20090528], DOI: 10.1016/J.MEHY.2009.04.023

## Description

### Technical Field

This invention relates to a composition comprising probiotic for use in preventing anxiety disorders. In particular, the probiotics are selected from the group consisting of strain Lactococcus lactis SL131, or of a combination of strains Bifidobacterium longum BL999 and Lactobacillus rhamnosus LPR, for use in preventing anxiety disorders in a mammal.

### Background of the Invention

Anxiety is a psychological and physiological state which results in an unpleasant feeling typically associated with uneasiness, fear, or worry. Anxiety is for example a normal reaction to stress. It may help a person to deal with a difficult situation at work or at school, but - when excessive - anxiety disorders result.

Anxiety disorders are a category of mental disorders characterized by feelings of anxiety and fear, the anxiety being a worry about future events and fear being a reaction to current events. These feelings may cause physical symptoms, such as a racing heart and shakiness. There are various forms of anxiety disorders, including generalized anxiety disorder, a specific phobia, social anxiety disorder, panic disorder, obsessive-compulsive disorder, post-traumatic stress disorder, separation anxiety and situational anxiety. While each of these anxiety disorders has its own characteristics and symptoms, they all include symptoms of anxiety.

Furthermore there appears to be a relationship between chronic stress, depression and anxiety. The chronic elevations in glucocorticoids (primarily cortisol) caused by excessive stressors in industrialised societies leads to actual physical changes in brain structure. Interestingly, emerging research suggests that psychoactive drugs, like those used in anxiety and depression, may stabilise mood not only by acting upon neurotransmitter levels but by modulating the action of glucocorticoids receptors in brain itself (Anacker C, Zunszain PA, Cattaneo LA, Garabedian MH, Thuret S et al., "Antidepressants increase human hippocampal neurogenesis by activating the glucocorticoid receptor", Mol. Psychiatry, 2011; 16: 738-750).

Children experience anxiety disorders similar to adults. The symptoms for the disorders are the same in children as they are in adults.

It is generally considered that anxiety disorders occur about twice as often in females as males, and generally begin during childhood.

Post-traumatic stress disorder is an anxiety disorder that results from a traumatic experience. It can result from an extreme situation, but also from long term (chronic) exposure to a severe stressor. It is usually a disorder which concerns adults.

Separation anxiety disorder is the feeling of excessive and inappropriate levels of anxiety of being separated from the person or place. Separation anxiety is a normal part of development in babies and children, and it is only when this feeling is excessive or inappropriate that it can be considered a disorder. Separation anxiety disorder is known as affecting roughly 7% of adults and 4% of children, the childhood cases tending to be more severe.

Common treatment options of anxiety disorders include lifestyle changes, therapy and medication. Medication is typically recommended only if other measures are not effective. Several methods of treatment have been found to be effective in treating childhood anxiety disorders. Children, like adults, may undergo psychotherapy, such as cognitive-behavioural therapy, or counselling. They may still be given medication as adults, but in much smaller doses. However, administering potent medication such as antidepressants to children is controversial. Moreover, children who have an anxiety disorder are likely to have other comorbid disorders such as depression, eating disorders, chronic stress and attention deficit disorders (both hyperactive and inattentive).

Prior art documents such as Laura Steenbergen et al «A randomized controlled trial to test the effect of multiplspecies probiotics on cognitive reactivity to sad mood» from Journal Brain, Behavior and Immunity in vol. 48, 1 Aug 2015; Bercik P et al "Chronic gastrointestinal inflammation induces anxiety-like behaviour and alters central nervous system biochemistry in mice" from Journal Gastroenterology in vol 139, no.6, 1 Dec 2010; Kantak Pranish A et al "Obsessive-compulsive-like behaviours in house mice are attenuated by a probiotc (Lactobacillus rhamnosus GG)" from Journal Behavioural Pharmacology in vol 25; patent documents such as EP 2110028; US 2015/305384 and US 2016/058808 have all discussed the subject-matter relevant to the present application.

Thus, there is a need to provide a composition which would help to prevent and/or treat anxiety disorders and related conditions like chronic stress, depression and mood regulation, in a mammal. This composition is particularly a nutritional composition for humans, more specifically selected from the group consisting of pregnant women, lactating women and children, even more preferably for pregnant women, lactating women or infants.

It is therefore an object of the invention to provide a composition for use in preventing anxiety disorders in a mammal, or at least to provide a useful alternative.

### Summary of the invention

The present inventors have found surprisingly that a composition is particularly effective for use in preventing anxiety disorders in a mammal.

Accordingly, in a first embodiment of the invention, there is provided a composition comprising probiotic selected from the group consisting of strain Lactococcus lactis SL131, or of a combination of strains Bifidobacterium longum BL999 and Lactobacillus rhamnosus LPR, for use in preventing anxiety, and wherein said mammal is the offspring of a mammal to whom said composition comprising probiotic has been administered during pregnancy and/or during lactation.

Surprisingly, the composition according to the invention improves synaptic flexibility by increasing the content of at least one of the specific proteins Arc (for Activity-Regulated Cytoskeleton-associated protein) and synaptophysin present in the hippocampus of the brain. These proteins allow better adaptation during fearful situations, help to modulate how fear is experienced during anxious situations and help prevent later fear in similar situations. These proteins are linked to each other, as there is some evidence that Arc upregulation causes synaptophysin expression to increase during the initial formation of fear conditioning (M. Mokin, J. S. Lindhal and J. Keifer, "Immediate-Early Gene-encoded protein Arc is associated with synaptic delivery of GluR4-containing AMPA receptors during in vitro classical conditioning" J. Neurophysiol 95 : 215-224, 2006).

The human is the offspring of a human mammal (woman) to whom said composition comprising probiotic has been administered.

### Detailed description of the invention

For a complete understanding of the present invention and the advantages thereof, reference is made to the following detailed description of the invention.

It should be appreciated that various embodiments of the present invention can be combined with other embodiments of the invention and are merely illustrative of the specific ways to make and use the invention, and do not limit the scope of the invention when taken into consideration with the claims and the following detailed description.

In the present description, the following words are given a definition that should be taken into account when reading and interpreting the description, examples and claims.

As used herein, the following terms have the following meanings.

The term "combination of compounds" means that said compounds are taken so that they can provide a synergistic effect in the organism. They can be taken simultaneously in the same mixture or taken at the same time or at different intervals of time so that they are present together in the organism to act synergistically.

The term "children" means humans between the stages of birth and puberty. An adult is a human older than a child.

The term "infant" means a child under the age of 12 months.

The term "preterm infant" (or "premature infant") means an infant born prior to 37 weeks gestational age.

The term "low birth weight infant" means an infant having a liveborn weight less than 2,500 g.

The term "infant formula" means a foodstuff intended for particular nutritional use by infants during the first four to six months of life and satisfying by itself the nutritional requirements of this category of person (Article 1.2 of the European Commission Directive 91/321/EEC of May 14, 1991 on infant formulae and follow-on formulae).

The term "preterm infant formula" means an infant formula intended for a preterm infant.

The term "human milk fortifier" means a supplement used to supplement the calories, protein, minerals and vitamins in breast milk fed to preterm infants or infants with a low birth weight.

The term "follow-on formula" means a foodstuff intended for particular nutritional use by infants aged over four months and constituting the principal liquid element in the progressively diversified diet of this category of person.

The term "starter infant formula" means a foodstuff intended for particular nutritional use by infants during the first four months of life.

The term "baby food" means a foodstuff intended for particular nutritional use by infants during the first years of life.

The term "infant cereal composition" means a foodstuff intended for particular nutritional use by infants during the first years of life.

The term "growing-up milk" means a milk-based beverage adapted for the specific nutritional needs of young children.

The term "Probiotic" means microbial cell preparations or components of microbial cells with a beneficial effect on the health or well-being of the host. (Salminen S, Ouwehand A. Benno Y. et al "Probiotics: how should they be defined" Trends Food Sci. Technol. 1999: 10 107-10). This definition of probiotic is generally accepted and considered to be in line with the WHO definition. The probiotic can comprise a unique strain of microorganism, of a mix of various strains and/or a mix of various bacterial species and genera. In case of combinations, the singular term "probiotic" can still be used to designate the probiotic combination or preparation. For the purpose of the present invention, microorganisms of the genus Lactococcus species lactis, microorganisms of the genus Bifidobacterium spaces longum and microorganisms of the genus Lactobacillus species rhamnosus are considered as probiotics.

The term "Prebiotic" means food substances intended to promote the growth of probiotic bacteria in the intestines.

The terms "Food grade" microorganisms are microorganisms that are safe for use in food.

The term "Edible" means a material that is approved for human or animal consumption.

The term "daily dosage" means the quantity to be taken in a day (24h), by the means of one or several doses.

As used in this specification, the words "comprises", "comprising", and similar words, are not to be interpreted in an exclusive or exhaustive sense. In other words, they are intended to mean "including, but not limited to".

Any reference to prior art documents in this specification is not to be considered an admission that such prior art is widely known or forms part of the common general knowledge in the field.

The composition for use according to the invention comprises probiotic selected from the group consisting of Lactococcus lactis SL131, and a combination of Bifidobacterium longum BL999 and Lactobacillus rhamnosus LPR, for use in preventing anxiety in a mammal.

In a first aspect, the invention relates to a composition comprising probiotic selected from the group consisting of strain Lactococcus lactis SL131, or of a combination of strains Bifidobacterium longum BL999 and Lactobacillus rhamnosus LPR, for use in preventing anxiety, and wherein said mammal is the offspring of a mammal to whom said composition comprising probiotic has been administered during pregnancy and/or during lactation.

The mammal is the offspring of a mammal to whom said composition comprising probiotic has been administered. The mammal and the offspring of a mammal may be human.

The anxiety disorders are usually generalized anxiety disorder, phobias, social anxiety disorder, panic disorder, obsessive-compulsive disorder, post-traumatic stress disorder, separation anxiety and situational anxiety. The related conditions are generally chronic stress, depression and mood regulation.

The mood disorders are usually recurrent or persistent depressive disorders e.g. depression, major depression, melancholic depression, atypical depression, minor depression, seasonal depression, bipolar affective disorder, dysthymia, menstrual cycle-related dysphoria, chronic fatigue syndrome, depression associated with somatoform disorders, postnatal depression or the baby blues, fibromyalgia and treatment-resistant depression

The composition for use in preventing anxiety according to the invention is preferably a nutritional composition for humans, more specifically selected from the group consisting of pregnant women, lactating women and children, even more preferably for pregnant women or lactating women or children in particular infants. Infants encompass preterm infants and low birth weight infants. In this later case, the nutritional composition is preferably an infant formula, a preterm infant formula, a human milk fortifier, a follow-on formula, a baby food, an infant cereal composition or a growing-up milk. It comprises a protein source, a source of available carbohydrates, a lipid source, wherein the lipid source comprises preferably DHA (docosahexaenoic acid), and more particularly the nutritional composition further comprises at least one prebiotic.

The composition is more suited for pregnant women and lactating women, or children prefrably infants. Actually, these groups are more prone and susceptible to stress. The hormonal equilibrium of a pregnant or lactating woman is different from its usual hormonal status (i.e. without being pregnant or lactating), which leads to an enhanced susceptibility to external factors. The importance of growth of foetuses and infants make their bodies more reactive to factors among which the equilibrium of their mother. Those groups can hence be more sensitive to the action of nutritional compositions, and in particular those nutritional compositions comprising ingredients acting on the nervous system and limiting the stress.

Of course, as known to the skilled person, these specific nutritional compositions comply with the nutritional requirements for the categories of persons concerned.

The composition for use in preventing anxiety disorders and/or mood disorders, and related conditions according to the invention preferably contains an amount of probiotic corresponding to 10⁴ to 10¹² cfu per daily dosage.

Probiotics can be provided as a pure form or can be incorporated in a matrix. Such matrix can advantageously protect the probiotics during the passage through the gastrointestinal tract (including the acidic conditions of the stomach) and enable live probiotics to arrive to the intestine. Such protective matrix can comprise sugar(s) (such as maltodextrin), proteins or fat component. In one embodiment the protective matrix comprises or is a vegetable oil.

Lactobacillus rhamnosus is LPR (CGMCC1.324).

It may be cultured according to any suitable method and prepared for encapsulation or addition to a nutritional composition by freeze-drying or spray-drying for example. Alternatively, it may be purchased already prepared in a suitable form for addition to food products.

Bifidobacterium longum BL 999 (or NCC 3001) was deposited under the Budapest treaty as ATCC BAA-999. BL999 may be obtained from Morinaga Milk Industry Co. Ltd. of Japan under the trade mark BB536.

It may be cultured according to any suitable method and prepared for encapsulation or addition to a nutritional composition by freeze-drying or spray-drying for example. Alternatively, it may be purchased already prepared in a suitable form for addition to food products.

Bifidobacterium longum and Lactobacillus rhamnosus may be administered separately or concurrently or in a mixture on their own, for example each one of the two of them enclosed in capsules each containing, for example, 10⁵ colony forming units (cfu) or incorporated in a nutritional composition such as a nutritionally complete formula (for example an infant formula or a clinical nutrition product), a dairy product, a beverage powder, a dehydrated soup, a dietary supplement, a meal replacement, a nutritional bar, a cereal, a confectionery product or a dry pet food. When incorporated in a nutritional composition, each of Bifidobacterium longum and Lactobacillus rhamnosus may be present in the composition in an amount corresponding to 10⁴ to 10¹², preferably 10⁷ to 10¹⁰ cfu/g (dry weight). Preferably Lactobacillus rhamnosus is present in an amount corresponding to 10⁵ to 10¹⁰, more preferably 10⁷ to 10¹⁰, cfu/g of dry composition and Bifidobacterium longum is present in an amount corresponding to 10⁵ to 10¹⁰, more preferably 10⁷ to 10¹⁰, cfu/g of dry composition.

Lactococcus lactis is SL131 (NCC 2287).

Lactobacoccus lactis may be administered on its own, for example enclosed in capsules each containing, for example, 10⁵ colony forming units (cfu) or incorporated in a nutritional composition such as a nutritionally complete formula (for example an infant formula or a clinical nutrition product), a dairy product, a beverage powder, a dehydrated soup, a dietary supplement, a meal replacement, a nutritional bar, a cereal, a confectionery product or a dry pet food. When incorporated in a nutritional composition, Lactococcus lactis may be present in the composition in an amount corresponding to between 10⁴ and 10¹² cfu/g (dry weight). Preferably Lactococcus lactis is present in an amount corresponding to 10⁵ to 10¹⁰, more preferably 10⁷ to 10¹⁰, cfu/g of dry composition.

It is preferred that at least a part of the probiotic, is alive in the composition and preferably arrive alive in the intestine. This way they can persist in the intestines and may increase the effectiveness by multiplication. They may also be effective by interacting with the commensal bacteria and/or the host.

Generally, the probiotic contained in the composition according to the invention will be administered in a prophylactic effective dose.

If said probiotic is present in a viable form, it is theoretically effective in any concentration considering the fact that these bacteria can colonize the gut and multiply.

These expressions of quantity of bacteria include the possibilities that the bacteria are live, inactivated or dead or even present as fragments such as DNA or cell wall materials or as metabolites. In other words, the quantities of bacteria are expressed in terms of the colony forming ability (cfu) of that quantity of bacteria as if all the bacteria were live irrespective of whether they are, in fact, live, inactivated or dead, fragmented or a mixture of any or all of these states.

The composition may be any composition, but is preferably a composition to be administered orally, enterally or rectally. For example, the composition may be an edible composition.

Typically, the composition may be selected from the group consisting of a nutritional composition, a pet food composition, a dietary supplement, a nutraceutical, a nutritional formula, a drink, and/or a medical composition.

If the composition for use according to the present invention is a nutritional composition, this has the advantage that such a composition can be distributed in pharmacies, drug stores, and also in normal supermarkets, where the compositions are easily available to everybody.

The generally pleasant taste of nutritional compositions will further contribute to the acceptance of the product. In particular small children or pets are much more likely to readily consume compositions with a taste that is generally liked.

Examples of nutritional compositions that are applicable to the present invention are yoghurts, milk, flavoured milk, ice cream, ready-to-eat desserts, powders for re-constitution with, e.g., milk or water, chocolate milk drinks, malt drinks, ready-to-eat dishes, instant dishes or drinks for humans or nutritional compositions representing a complete or a partial diet intended for pets or livestock.

Consequently, in one embodiment, the composition according to the present invention is a nutritional composition intended for use by humans, pets or livestock. The terms "intended for use by" means that there are specifically adapted for the nutritional needs of the targeted mammalian population. The skilled person is aware of the ingredients useful for such nutritional compositions, in addition to the probiotic according to the invention, so as to make them suitable as supplements or nutritionally complete composition.

The composition according to the present invention is for use in animals selected from the group consisting of dogs, cats, pigs, cattle, horses, goats, sheep or poultry.

In a preferred embodiment encompassed in the wordings of the claims of the present invention, the composition is a nutritional composition for pregnant women and lactating women. In this case, as illustrated in the following examples, the foetus or the breastfed child is likely to be indirectly exposed to the probiotics by being in contact with the mother and through breast milk.

The composition comprising probiotic, as defined herein, is for use in preventing anxiety disorders in a mammal, wherein said mammal is the offspring of a mammal to whom said composition comprising probiotic has been administered. Said composition comprising probiotic is administered to the mammal during pregnancy and/or during lactation. Preferably, said composition is administered to a human mammal (woman) in at least the 2nd and/or 3rd trimesters of pregnancy and/or during lactation for up to 12 months postnatally.

The composition is administered to a pregnant mammal and/or to a lactating mammal. For example the mammal may be human and the composition may be administered to a pregnant woman and/or a lactating women.

If the composition is administered to a pregnant or lactating woman, the composition may be administered from at least 4, at least 8, at least 12, at least 16, at least 20, at least 24, at least 28, at least 36 weeks of pregnancy.

Since in humans infants synaptogenesis starts in-utero in the 2nd semester of pregnancy, administration of the composition may be particularly advantageous in the 2nd and/or 3rd semester of pregnancy. Further, since synaptogenesis continues postnatally administration to the woman during lactation may be particularly advantageous e.g. up to 18mths postpartum during lactation e.g. up to 12months, up to 9months, up to 8, 7, 6, 5, 4, 3, 2, 1 month postpartum during lactation.

Anxiety and/or mood disorders, and related disorders, may be more prevalence in the offspring of mothers who have suffered from anxiety disorders or related conditions, in particular during pregnancy or the breastfeeding period (lactation). Accordingly administration of the composition comprising probiotic, as defined herein, may be particularly beneficial in mothers who suffer from anxiety disorders and/or mood disorders or related disorders.

The composition may also be used to promote support or optimise synaptogenesis in a mammal.

The composition may for example be used to promote support or optimise synaptogenesis in a mammal, wherein said mammal is the offspring of a mammal to whom said composition comprising probiotic has been administered.

The composition may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents) , flowing agents, taste masking agents, weighting agents, jellifying agents, gel forming agents, antioxidants and antimicrobials. The composition may also contain conventional pharmaceutical additives and adjuvants, excipients and diluents, including, but not limited to, water, gelatine of any origin, vegetable gums, ligninsulfonate , talc, sugars, starch, gum arabic, vegetable oils, polyalkylene glycols, flavouring agents, preservatives, stabilizers, emulsifying agents, buffers, lubricants, colorants, wetting agents, fillers, and the like. In all cases, such further components will be selected having regard to their suitability for the intended recipient.

The composition may comprise a source of protein.

Any suitable dietary protein may be used, for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred.

The proteins may be intact or hydrolysed or a mixture of intact and hydrolysed proteins. It may be desirable to supply partially hydrolysed proteins (degree of hydrolysis between 2 and 20%), for example for human subjects and/or animals at risk of developing cows' milk allergy.

Furthermore, pre-hydrolysed protein sources are generally more easily digested and absorbed by an impaired gastro-intestinal tract.

If hydrolysed proteins are required, the hydrolysis process may be carried out as desired and as is known in the art. It may be desirable to supply partially hydrolysed proteins (degree of hydrolysis between 2 and 20%).

For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

The composition may also contain a source of carbohydrates and a lipid source.

If the composition includes a lipid source, the lipid source preferably provides 5% to 40% of the energy of the composition; for example 20% to 30% of the energy. A suitable lipid profile may be obtained using a blend of canola oil, corn oil and high-oleic acid sunflower oil.

A source of carbohydrate may be added to the composition.

The source of carbohydrates preferably provides 40% to 80% of the energy of the composition. Any suitable carbohydrate may be used, for example sucrose, lactose, glucose, fructose, corn syrup solids, maltodextrins, and mixtures thereof. Dietary fibre may also be added if desired. Dietary fibre passes through the small intestine undigested by enzymes and functions as a natural bulking agent and laxative. Dietary fibre may be soluble or insoluble and in general a blend of the two types is preferred. Suitable sources of dietary fibre include soy, pea, oat, pectin, guar gum, partially hydrolysed guar gum, gum Arabic, fructooligosaccharides, acidic oligosaccharides, galacto-oligosaccharides, sialyl-lactose and oligosaccharides derived from animal milks. A preferred fibre blend is a mixture of inulin with shorter chain fructo- oligosaccharides. Preferably, if fibre is present, the fibre content is between 2 and 40 g/ml of the composition as consumed, more preferably between 4 and 10 g/l.

The composition may also contain minerals and micronutrients such as trace elements and vitamins in accordance with the recommendations of government bodies such as the USRDA. For example, the composition may contain per daily dosage one or more of the following micronutrients in the ranges given: 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 µg iodine, 5 to 15 µg selenium, 1000 to 3000 µg beta carotene, 10 to 80 mg vitamin C, 1 to 2 mg vitamin B1, 0.5 to 1.5 mg vitamin B6, 0.5 to 2 mg vitamin B2, 5 to 18 mg niacin, 0.5 to 2.0 µg vitamin B12, 100 to 800 µg folic acid, 30 to 70 µg biotin, 1 to 5 µg vitamin D, 3 to 10 µg vitamin E.

One or more food grade emulsifiers may be incorporated into the composition if desired; for example diacetyl tartaric acid esters of mono- and di-glycerides, lecithin and mono- and di-glycerides. Similarly suitable salts and stabilisers may be included.

The composition may be orally and/or enterally administrable; for example in the form of a powder for re-constitution with milk or water.

The composition may comprise at least one other kind of other food grade microorganism.

The food grade microorganisms are preferably food-grade bacteria or food-grade yeast. The food grade bacteria may be selected from the group consisting of lactic acid bacteria, bifidobacteria, propionibacteria or mixtures thereof. As food grade yeast for example Saccharomyces cerevisiae and/or Saccharomyces boulardii can be used.

The composition further may contain at least one prebiotic. Prebiotics can thus promote the growth of certain food grade bacteria, in particular of probiotic bacteria, in the intestines and can hence enhance the effect of the probiotic contained in the composition according to the invention. Furthermore, several prebiotics have a positive influence on, e.g., digestion.

Preferably the prebiotic may be selected from the group consisting of oligosaccharides and optionally contain fructose, galactose, mannose, soy and/or inulin; dietary fibres; or mixtures thereof. The composition of the present invention may be provided in powder form having a water activity less than 0.2, for example in the range of 0.19-0.05, preferably less than 0.15.

The composition may be a shelf stable powder. The low water activity provides this shelf stability and ensures that probiotic will remain viable even after long storage times.

Water activity or Aw is a measurement of the energy status of the water in a system. It is defined as the vapour pressure of water divided by that of pure water at the same temperature; therefore, pure distilled water has a water activity of exactly one.

The advantages, nature, and various additional features of the invention will appear more fully upon consideration of the illustrative experiments now to be described in detail in connection with accompanying drawings.

In the drawings:
FIG. 1 is a bar graph plotting the results of the experiments, in terms of total seconds (s) spent in a light box with respect to control mice ("con") and mice treated with the probiotic Lactococcus lactis (SL131) ("pro (SL131)").
FIG. 2 is a bar graph plotting the results of the experiments, in terms of Arc mRNA (normalised to beta-actin expression) with respect to control mice ("con") and mice treated with the probiotic Lactococcus lactis (SL131) ("pro (SL131)").

The invention is further described with reference to the following examples. It will be appreciated that the invention as claimed is not intended to be limited in any way by these examples.

### Examples 1 and 2: use of Lactococcus lactis SL131

### Bacterial culture conditions:

Probiotic: Lactococcus lactis SL131 (NCC 2287). It was grown in industrially adapted growth medium. Cells were harvested 3h after reaching the stationary phase. Biomass was then collected and dried using standard drying methods.

### Animals:

The tested animals were female BALB/CByj and their offspring. Females were ordered from Charles River shortly after being impregnated.

Pregnancy was established by the supplier by verifying the presence of vaginal plugs. Mothers and offspring, born in the animal facility of the Laboratories, were marked with tattoo ink on their tail.

The pregnant females were housed in individual cages. Shortly after the birth, mother and offspring mice were removed to a separate cage, with two offspring mice (of the same gender) per cage.

The pregnant mothers were separated in two groups, each receiving from the day of their arrival in the facilities a standard rodent diet, one group (A) not receiving any probiotic (maltodextrin in drinking water instead of probiotic); and another group (B) receiving Lactococcus lactis SL131 via drinking water at 5.10⁸ cfu/mL (the drinking water being refreshed daily) from embryonic day 5 (E5) to post-natal day 14 (PND 14). During the last week of lactation prior to weaning (PND 15-21), SL131 was administered by gavage (5.10⁹ cfu/0.15mL) to the mothers. Offspring were exposed to SL131 as foetuses and also as newborn pups via breast milk. Offspring were weaned on PND 21 and selected groups of offspring were allowed to grow to maturity (10 weeks of age, at which point they were given a light/dark box test.) Three days after the light/dark box testing, they were sacrificed and brain extracted for microarray analysis. Mice used for testing: 10 pups from n=5 "control" mothers and 21 pups from n=11 "SL131" mothers (mothers that received SL131 in drinking water during pregnancy and lactation).

### Example 1: Anxiety behaviour (light/dark box) - use of Lactococcus lactis SL131

### Light/dark box:

Anxiety behaviour was assessed individually in mice using a light box / dark box (or light/dark box) as described in the literature. Briefly, each mouse was placed in the centre of an illuminated bright box (30x30 cm) (called light box) connected by an opening (10x3 cm) to a smaller dark box (30x15 cm). The locomotor behaviour of each mouse in the light box was recorded for 5 minutes by a digital video camera and stored in a computer for an off-line analysis. The total time spent in light box was assessed.

The p value of <0.05 was considered as statistically significant.

### Results:

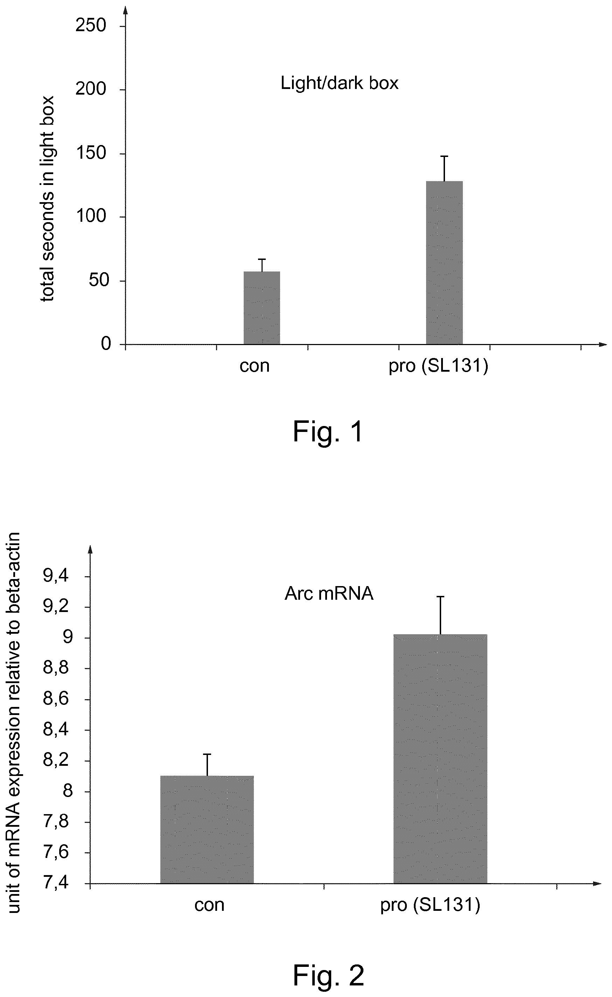

As illustrated in figure 1, mice treated with Lactococcus lactis SL131 showed an increase in the time spent in the light box with respect to the control animals (p<0.05). Treatment with Lactococcus lactis SL131 induced a reduction of anxiety-like behaviour towards normality.

### Example 2: Arc protein content in hippocampus - use of Lactobacillus lactis SL131

Arc protein is an important transcription factor for synaptic plasticity, where upregulation of Arc transcription has been associated with improved memory, synaptic plasticity and decreased anxiety (Bramham CR, Worley PF, Moore MJ, Guzowski JF. "The immediate early gene arc/arg3.1: regulation, mechanisms, and function" J. Neurosci. 2008 Nov 12; 28(46): 11760-7). Arc protein is found in neuronal axons and dendrites and local synaptic activity increase Arc in a time dependent manner (Rodriguez J, Davies H, Silva A, De Souza I, Peddie C et al. "Long-term potentiation in the rat dentate gyrus is associated with enhanced Arc/Arg3.1 protein expression in spines, dendrites and glia" European Journal of Neuroscience, Vol. 21, pp. 2384-2396, 2005). These unique features implied a function for Arc in coupling synaptic activity to protein synthesis-dependent synaptic plasticity.

Robust arc expression in the hippocampus appears to protect individuals from anxiety and stress. Kozlovsky et al. (Kozlovsky N, Matar MA, Kaplan Z, Kotler M, Zohar J, Cohen H. "The immediate early gene Arc is associated with behavioral resilience to stress exposure in an animal model of post-traumatic stress disorder" Eur Neuropsychopharmacol. 2008 Feb;18(2):107-16) reported that, among animals exposed to stressful events during development, individuals that showed resilience and decreased stress response had high Arc. Moreover, decreased Arc hippocampal expression in rats has been shown to be correlated to later higher rates of post-traumatic stress disorder behaviour while high Arc expression appears to be protective against stress (Nalloor R, Bunting KM, Vazdarjanova A. "Altered hippocampal function before emotional trauma in rats susceptible to PTSD-like behaviors" Neurobiol Learn Mem. 2014 Jul; 112:158-6). Interestingly, hippocampal Arc is also implicated for protection against depression, and antidepressants have been shown to robustly increase Arc (Eriksson TM, Delagrange P, Spedding M, Popoli M, Mathé AA, Ögren SO, Svenningsson P. "Emotional memory impairments in a genetic rat model of depression: involvement of 5-HT/MEK/Arc signaling in restoration" Mol Psychiatry. 2012 Feb;17(2):173-84; Wibrand K, Berge K, Messaoudi M, Duffaud A, Panja D, Bramham CR, Burri L. "Enhanced cognitive function and antidepressant-like effects after krill oil supplementation in rats" Lipids Health Dis. 2013 Jan 25;12:6).

### Microarray Method

Three days after offspring were given the light dark box test, they were euthanised and brain extracted for microarray analysis. Mice used for post mortem analysis: n=6 pups from "control" mothers and n=6 pups from mothers receiving SL131. Hippocampal tissue was excised and stored at -80° C and total RNA was extracted following the supplier's protocol.

RNA from 6 mice of the control group and 6 mice from the SL131-treated group were used for microarray analysis. Total RNA was extracted and purified with the RNAdvance tissue kit (Agencourt, Beverly, MA, USA). The quality of RNA samples was checked by using the Fragment Analyser (Advanced Analytical Technologies, Inc, Ames, IA, USA). All cRNA targets were synthesized, labelled, and purified according to the Illumina TotalPrep RNA amplification protocol (Applied Biosystems, Austin, TX, USA). Then, 15 µl of each hybridization mix was dispensed on the microarrays (16 h, 58°C), the microarrays were washed to remove non hybridized material and stained with Streptavidin-Cy3. All samples were analysed with the microarrays MouseRef-8 v2 Expression BeadChips (Illumina, San Diego, CA, USA). Microarray analysis was performed by the Nestle Institute of Health Sciences Genomics core.

### Results:

The microarray revealed, as illustrated in Figure 2 (unit of mRNA expression relative to beta-actin), significant differences between the control and SL131-treated hippocampal tissue in activity-related cytoskeletal-associated protein (Arc) transcription (p<0.0008).

### Example 3: Synaptophysin protein content in hippocampus - use of a combination of Lactobacillus rhamnosus LPR and Bifidobacterium longum SL999

### Material and methods:

Probiotic: Bifidobacterium longum BL999 (ATCC BAA-9999) and Lactobacillus rhamnosus LPR (ATCC 54193) in powdered form.

Tests were conducted on whether maternal dietary supplementation with probiotics during pregnancy and lactation affects the synaptophysin level in the hippocampus of their offspring. The tested animals were female BALB/CByj and their offspring. Females were ordered from Charles River shortly after being impregnated.

Pregnancy was established by the supplier by verifying the presence of vaginal plugs. Mothers and offspring, born in the animal facility of the Laboratories, were marked with tattoo ink on their tail.

The pregnant females were housed in individual cages. Shortly after the birth, mother and offspring mice were removed to a separate cage, with two offspring mice (of the same gender) per cage.

The pregnant mothers were separated in two groups, each receiving from the day of their arrival in the facilities a standard rodent diet, one group (A) not receiving any probiotic (maltodextrin in drinking water instead of probiotic); and another group (B) receiving a mixture of Bifidobacterium longum BL 999 and Lactobacillus rhamnosus LPR in drinking water (5.10⁸ cfu/mL of each bacteria, the drinking water being refreshed daily) from embryonic day 5 (E5) to post-natal day 14 (PND 14). During the last week of lactation prior to weaning (PND 15-21), probiotic were administered by gavage (5.10⁹ cfu/0.15mL of each bacteria) to the mothers. Offspring were exposed to probiotic as foetuses and also as newborn pups via breast milk. Offspring were weaned on PND 21 and selected groups of offspring were allowed to grow to maturity (at least 10 weeks of age). Offspring mice were euthanatized at week 13.

### Results:

The following Table 1 provides is a summary of statistics of Synaptophysin in offspring mice euthanatized at 13 weeks.

**Table 1**

| Variable | Group | N | Mean | SD | SE Mean | Median | SDrobust | SE Median | Min | Max |
|---|---|---|---|---|---|---|---|---|---|---|
| Synaptophysin relative value | A - No probiotics | 7 | 11.8 | 0.7 | 0.26 | 12.16 | 0.76 | 0.36 | 10.72 | 12.56 |
| | B - Bl999 & LPR | 7 | 13.86 | 1.8 | 0.68 | 14.35 | 1.9 | 0.9 | 11.54 | 16.38 |

Wherein N is the number of offspring, Mean is the mean value of Synaptophysin relative value to beta-actin, a house-keeping protein (normalised to beta-actin expression), SD is the Standard Deviation, SE Mean is the Standard Error of the Mean, SD_{robust} is the Robust Standard deviation, SE Median is the Standard Error of the Median based on SD_{robust}, Min is the minimum value and Max is the maximum value.

Following table 2 shows the results of statistic tests (median of the reference group, estimate and percentage change from the median, Hodges-Lehmann (HL) estimate, 95% Confidence Interval (CI), p value) of the comparisons of interest in terms of Synaptophysin of offspring mice euthanatized at 13 weeks.

**Table 2**

| **Outcome** | **Comparison** | **Median A - No probiotics** | **HL Estimate (% change)** | **95% CI Lower** | **95% CI Upper** | **P-value** |
|---|---|---|---|---|---|---|
| Synaptophysin in offspring mice euthanatized at 13 weeks | B - Bl999 & LPR vs. A - No probiotics | 12.16 | 2.21 (18.19%) | -0.12 | 3.86 | 0.053 |

In conclusion, group B according to the invention revealed significantly higher values of synaptophysin than control group A.

Conclusion of the experiments disclosed in examples 1, 2 and 3: Probiotics were administered during pregnancy/breastfeeding. The probiotic treatment had been stopped 2 months before the tests and yet still showed behavioral effects. A difference was observed in anxiety, which difference was unrelated to previous stress exposure or disease modeling (ie.. colitis etc). Thus probiotics proved to provide a neurodevelopmental effect in preventing and/or treating anxiety disorders and related conditions.

Although the invention has been described by way of example, it should be appreciated that variations and modifications may be made without departing from the scope of the invention as defined in the claims. Furthermore, where known equivalents exist to specific features, such equivalents are incorporated as if specifically referred to in this specification.

## Claims

1. A composition comprising probiotic selected from the group consisting of strain Lactococcus lactis SL131, or of a combination of strains Bifidobacterium longum BL999 and Lactobacillus rhamnosus LPR, for use in preventing anxiety disorders, and wherein said mammal is the offspring of a mammal to whom said composition comprising probiotic has been administered during pregnancy and/or during lactation.

2. The composition for use according to claim 1, wherein Lactobacillus rhamnosus is present in an amount corresponding to 10⁵ to 10¹⁰ cfu/g of dry composition and Bifidobacterium longum is present in an amount corresponding to 10⁵ to 10¹⁰ cfu/g of dry composition.

3. The composition for use according to any one of claims land 2, wherein Lactobacillus rhamnosus is present in an amount corresponding to 10⁷ to 10¹⁰ cfu/g of dry composition and Bifidobacterium longum is present in an amount corresponding to 10⁷ to 10¹⁰ cfu/g of dry composition.

4. The composition for use according to claims 1, wherein the probiotic is Lactococcus lactis SL131.

5. The composition for use according to claim 4, wherein Lactococcus lactis is present in an amount corresponding to 10⁵ to 10¹⁰ cfu/g of dry composition.

6. The composition for use according to any one of claims 4 and 5, wherein Lactococcus lactis is present in an amount corresponding to 10⁷ to 10¹⁰ cfu/g of dry composition.

7. The composition for use according to any one of the preceding claims, wherein anxiety disorders are generalized anxiety disorder, phobias, social anxiety disorder, panic disorder, obsessive-compulsive disorder, post-traumatic stress disorder, separation anxiety and situational anxiety.

8. The composition for use according to any one of the preceding claims, wherein the composition is a nutritional composition for humans.

9. The composition for use according to claim 8, wherein the composition is a nutritional composition for pregnant women and lactating women.

10. The composition for use according to claim 9, wherein said composition is administered to the pregnant mammal in at least the 2^{nd} and/or 3^{rd} trimesters of pregnancy.

11. The composition for use according to claim 9 or 10, wherein said composition is administered to the lactating mammal during lactation for up to 12 months postnatally.

## Patentansprüche

1. Zusammensetzung, umfassend ein Probiotikum, ausgewählt aus der Gruppe bestehend aus Stamm Lactococcus lactis SL131, oder aus einer Kombination von Stämmen Bifidobacterium longum BL999 und Lactobacillus rhamnosus LPR, zur Verwendung bei der Vorbeugung von Angststörungen, und wobei das Säugetier der Nachwuchs eines Säugetiers ist, dem die Zusammensetzung, umfassend Probiotikum während der Schwangerschaft und/oder während der Laktation verabreicht worden ist.

2. Zusammensetzung zur Verwendung nach Anspruch 1, wobei Lactobacillus rhamnosus in einer Menge entsprechend 10⁵ bis 10¹⁰ KBE/g an trockener Zusammensetzung vorliegt und Bifidobacterium longum in einer Menge entsprechend 10⁵ bis 10¹⁰ KBE/g an trockener Zusammensetzung vorliegt.

3. Zusammensetzung zur Verwendung nach einem der Ansprüche 1 und 2, wobei Lactobacillus rhamnosus in einer Menge entsprechend 10⁷ bis 10¹⁰ KBE/g an trockener Zusammensetzung vorliegt und Bifidobacterium longum in einer Menge entsprechend 10⁷ bis 10¹⁰ KBE/g an trockener Zusammensetzung vorliegt.

4. Zusammensetzung zur Verwendung nach Anspruch 1, wobei das Probiotikum Lactococcus lactis SL131 ist.

5. Zusammensetzung zur Verwendung nach Anspruch 4, wobei der Lactococcus lactis in einer Menge entsprechend 10⁵ bis 10¹⁰ KBE/g an trockener Zusammensetzung vorliegt.

6. Zusammensetzung zur Verwendung nach einem der Ansprüche 4 und 5, wobei der Lactococcus lactis in einer Menge entsprechend 10⁷ bis 10¹⁰ KBE/g an trockener Zusammensetzung vorliegt.

7. Zusammensetzung zur Verwendung nach einem der vorstehenden Ansprüche, wobei die Angststörungen generalisierte Angststörung, Phobien, Störung mit sozialer Angst, Panikstörung, Zwangsstörung, posttraumatische Belastungsstörung, Trennungsangst und situative Angst sind.

8. Zusammensetzung zur Verwendung nach einem der vorstehenden Ansprüche, wobei die Zusammensetzung eine Nährstoffzusammensetzung für Menschen ist.

9. Zusammensetzung zur Verwendung nach Anspruch 8, wobei die Zusammensetzung eine Nährstoffzusammensetzung für schwangere Frauen und stillende Frauen ist.

10. Zusammensetzung zur Verwendung nach Anspruch 9, wobei die Zusammensetzung dem schwangeren Säugetier mindestens im 2^{ten} und/oder 3^{ten} Trimester der Schwangerschaft verabreicht wird.

11. Zusammensetzung zur Verwendung nach Anspruch 9 oder 10, wobei die Zusammensetzung dem laktierenden Säugetier während der Laktation für bis zu 12 Monate postnatal verabreicht wird.

## Revendications

1. Composition comprenant un probiotique choisi dans le groupe constitué de la souche Lactococcus lactis SL131, ou d'une combinaison des souches Bifidobacterium longum BL999 et Lactobacillus rhamnosus LPR, pour utilisation dans la prévention des troubles anxieux, et dans laquelle ledit mammifère est la progéniture d'un mammifère à qui ladite composition comprenant un probiotique a été administrée pendant la grossesse et/ou pendant l'allaitement.

2. Composition pour utilisation selon la revendication 1, dans laquelle le Lactobacillus rhamnosus est présent en une quantité correspondant à 10⁵ à 10¹⁰ ufc/g de composition sèche et le Bifidobacterium longum est présent en une quantité correspondant à 10⁵ à 10¹⁰ ufc/g de composition sèche.

3. Composition pour utilisation selon l'une quelconque des revendications 1 et 2, dans laquelle le Lactobacillus rhamnosus est présent en une quantité correspondant à 10⁷ à 10¹⁰ ufc/g de composition sèche et le Bifidobacterium longum est présent en une quantité correspondant à 10⁷ à 10¹⁰ ufc/g de composition sèche.

4. Composition pour utilisation selon la revendication 1, dans laquelle le probiotique est le Lactococcus lactis SL131.

5. Composition pour utilisation selon la revendication 4, dans laquelle le Lactococcus lactis est présent en une quantité correspondant à 10⁵ à 10¹⁰ ufc/g de composition sèche.

6. Composition pour utilisation selon l'une quelconque des revendications 4 et 5, dans laquelle le Lactococcus lactis est présent en une quantité correspondant à 10⁷ à 10¹⁰ ufc/g de composition sèche.

7. Composition pour utilisation selon l'une quelconque des revendications précédentes, dans laquelle les troubles anxieux sont le trouble anxieux généralisé, les phobies, le trouble d'anxiété sociale, le trouble panique, le trouble obsessionnel compulsif, le trouble de stress post-traumatique, l'anxiété de séparation et l'anxiété situationnelle.

8. Composition pour utilisation selon l'une quelconque des revendications précédentes, dans laquelle la composition est une composition nutritionnelle pour humains.

9. Composition pour utilisation selon la revendication 8, dans laquelle la composition est une composition nutritionnelle pour femmes enceintes et femmes qui allaitent.

10. Composition pour utilisation selon la revendication 9, dans laquelle ladite composition est administrée au mammifère en état de grossesse au cours au moins du 2^{ième} et/ou 3^{ième} trimestres de grossesse.

11. Composition pour utilisation selon la revendication 9 ou 10, dans laquelle ladite composition est administrée au mammifère qui allaite pendant l'allaitement jusqu'à 12 mois après la naissance.
